(19) Europäisches Patentamt
European Patent Office
Office européen des brevets

(11) Numéro de publication: **0 187 551 A1**

(12) **DEMANDE DE BREVET EUROPEEN**

(21) Numéro de dépôt: 85400024.7

(22) Date de dépôt: 08.01.85

(51) Int. Cl.⁴: C 07 D 487/04
C 07 D 491/14, A 61 K 31/50
C 07 D 237/28
//(C07D487/04, 237:00, 231:00)

(43) Date de publication de la demande: 16.07.86 Bulletin 86/29

(84) Etats contractants désignés: AT BE CH DE FR GB IT LI LU NL SE

(71) Demandeur: ROUSSEL-UCLAF
35, boulevard des Invalides
F-75007 Paris(FR)

(72) Inventeur: Gasc, Jean-Claude
6, place Georges Lyssandre
F-93140 Bondy(FR)

(72) Inventeur: Humbert, Daniel
15, rue Gaston Charle
F-94120-Fontenay-Sous-Bois(FR)

(72) Inventeur: Hunt, Peter Francis
18, rue de LHotel Dieu
F-95500 Gonesse(FR)

(74) Mandataire: Vieillefosse, Jean-Claude et al,
Département des Brevets ROUSSEL UCLAF B.P no 9
F-93230 Romainville(FR)

(54) **Dérivés de la pyrazolo/4,3-c/cinnolin-3-one, leurs sels, procédé de préparation, application à titre de médicaments, compositions les renfermant et produits intermédiaires.**

(57) L'invention concerne les dérivés de la pyrazolo /4,3-c/ cinnolin-3-one ainsi que leurs sels d'addition avec les acides minéraux ou organiques, de formule:

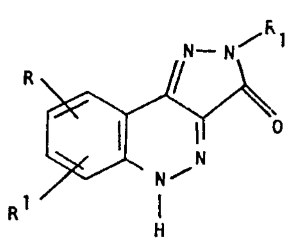

dans laquelle R en position 7 ou 8 = halogène, alkyle linéaire ou ramifié $C_1$–$C_5$, alkoxy, nitro ou trifluorométhyle ou R + R' = pont alkylène $C_1$–$C_4$–dioxy
R' = H ou R' + R = pont alkylène $C_1$–$C_4$ dioxy
$R_1$ = phényle, pyridyle, thiazolyle, dihydrothiazolyle ou thiényle éventuellement substitués, leur procédé de préparation, leur application à titre de médicaments, les compositions les renfermant et les produits intermédiaires.

EP 0 187 551 A1

Dérivés de la pyrazolo/4,3-c/cinnolin-3-one, leurs sels, procédé de préparation, application à titre de médicaments, compositions les renfermant et produits intermédiaires.

La présente invention concerne de nouveaux dérivés de la pyrazolo /4,3-c/ cinnolin-3-one ainsi que leurs sels, le procédé de préparation, l'application à titre de médicaments de ces nouveaux dérivés, les compositions les renfermant et les produits intermédiaires obtenus.

L'invention a pour objet de nouveaux dérivés de la pyrazolo/4,3-c/ cinnolin-3-one, ainsi que leurs sels d'addition avec les acides minéraux ou organiques, caractérisés en ce qu'ils répondent à la formule générale (I) :

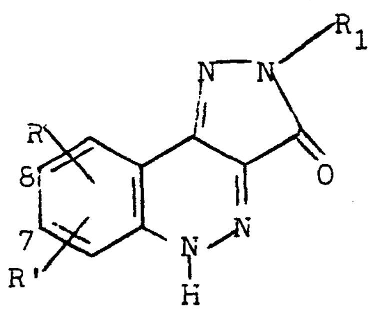

(I)

dans laquelle R en position 7 ou 8 représente un atome d'hydrogène, un atome d'halogène, un radical alkyle, linéaire ou ramifié renfermant de 1 à 5 atomes de carbone, un radical alkoxy renfermant de 1 à 5 atomes de carbone, un radical nitro ou trifluorométhyle, ou ensemble avec R' forme un pont alkylène $C_1$-$C_4$ dioxy en 7-8, R' représente un atome d'hydrogène ou ensemble avec R représente un pont alkylène $C_1$-$C_4$ dioxy en 7-8, et $R_1$ représente un radical phényle, pyridyle, thiazolyle, dihydrothiazolyle ou thiényle, éventuellement substitués par un radical alkyle, linéaire ou ramifié, renfermant de 1 à 5 atomes de

carbone, par un radical alkoxy renfermant de 1 à 5 atomes de carbone, par un atome d'halogène, par un groupement mono, di- ou tri-halogéno méthyle, ou par un groupement aralkyloxy renfermant de 7 à 12 atomes de carbone.

Dans la formule générale (I) et dans ce qui suit, lorsque R représente un atome d'halogène, il s'agit, par exemple, d'un atome de brome ou d'iode et de préférence, un atome de chlore ; le terme radical alkyle linéaire renfermant de 1 à 5 atomes de carbone désigne, par exemple, un radical butyle ou pentyle, et de préférence un radical méthyle, éthyle ou n-propyle et dans le cas d'un radical alkyle ramifié, ce radical comporte de 3 à 5 atomes de carbone et désigne, de préférence, un radical isopropyle ou isobutyle ; le terme radical alkoxy renfermant de 1 à 5 atomes de carbone désigne, par exemple, un radical butoxy ou pentoxy et de préférence un radical méthoxy, éthoxy ou propoxy ; le pont alkylène $C_1$-$C_4$ dioxy est, par exemple, un pont propylène ou butylène-dioxy, mais de préférence méthylène ou éthylène dioxy, le mono-, di- ou tri-halogénométhyle peut être par exemple le bromométhyle, le chloro ou dichlorométhyle et de préférence, le trifluorométhyle, le groupement aralkyloxy est par exemple un groupement naphtylméthyloxy et de préférence un groupement benzyloxy.

Les sels d'addition avec les acides minéraux ou organiques peuvent être, par exemple, les sels formés avec les acides chlorhydrique, bromhydrique, nitrique, sulfurique, phosphorique, acétique, formique, propionique, benzoïque, maléïque, fumarique, succinique, tartrique, citrique, oxalique, glyoxylique, aspartique, alcane sulfoniques tels que les acides méthane ou éthane sulfoniques, arylsulfoniques, tels que les acides benzène ou paratoluène sulfoniques et arylcarboxyliques.

Parmi les produits, objet de l'invention, on peut citer notamment les dérivés répondant à la formule (I) ci-dessus, ainsi que leurs sels d'addition avec les acides minéraux ou organiques, caractérisés en ce que, dans ladite formule (I), R en position 7 ou 8 représente un atome d'hydrogène, un atome d'halogène, un radical alkyle, linéaire ou ramifié renfermant de 1 à 5 atomes de carbone, un radical alkoxy renfermant de 1 à 5 atomes de carbone, un radical nitro ou trifluorométhyle, R' représente un atome d'hydrogène et $R_1$ représente un radical phényle éventuellement substitué par un radical alkyle linéaire ou ramifié renfermant de 1 à 5 atomes de carbone, par un radical alkoxy renfermant de 1 à 5 atomes de carbone ou par un atome d'halogène ou $R_1$ représente un radical pyridyle ou thiazolyle.

Parmi ceux-ci, on peut citer plus particulièrement les dérivés

caractérisés en ce que, dans ladite formule (I), R se trouve en position 8 et $R_1$ représente un radical phényle ainsi que leurs sels d'addition avec les acides minéraux ou organiques, et tout particulièrement la 8-chloro 2,5-dihydro 2-phényl 3H-pyrazolo/4,3-c/ cinnolin-3-one, la 2,5-dihydro 2-phényl 3H-pyrazolo/4,3-c/ cinnolin-3-one et la 2,5-dihydro 2-pyrid-2-yl 3H-pyrazolo/4,3-c/ cinnolin-3-one ainsi que ses sels d'addition avec les acides minéraux ou organiques.

On peut citer également :

- la 2,5-dihydro 2-pyrid-3-yl 3H-pyrazolo/4,3-c/ cinnolin-3-one,
- la 2,5-dihydro 2-pyrid-4-yl 3H-pyrazolo/4,3-c/ cinnolin-3-one,
- la 2-(4-chlorophényl)2,5-dihydro 3H-pyrazolo/4,3-c/ cinnolin-3-one,
- la 2,5-dihydro 8-méthoxy (2-phényl) 3H-pyrazolo/4,3-c/ cinnolin-3-one,
- la 2,5-dihydro 8-méthoxy 2-pyrid-2-yl 3H-pyrazolo/4,3-c/ cinnolin-3-one,
- la 2,5-dihydro 7,8-méthylène dioxy 2-pyrid-2-yl 3H-pyrazolo/4,3-c/ cinnolin-3-one et
- la 2,5-dihydro 7,8-méthylène dioxy 2-phényl 3H-pyrazolo/4,3-c/ cinnolin-3-one ainsi que leurs sels d'addition avec les acides minéraux ou organiques.

L'invention a également pour objet un procédé de préparation des dérivés, tels que définis par la formule (I) ci-dessus, ainsi que de leurs sels, caractérisé en ce que l'on fait réagir un produit de formule (II) :

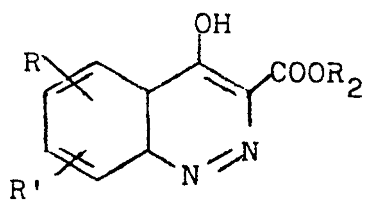

(II)

dans laquelle R et R' ont la signification déjà indiquée et $R_2$ représente un radical alkyle linéaire renfermant de 1 à 5 atomes de carbone, avec un agent de chloration, pour obtenir un produit de formule (III) :

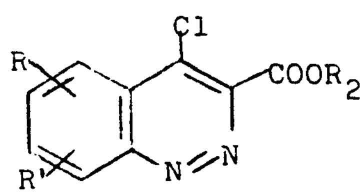

(III)

dans laquelle R, R' et $R_2$ ont la signification déjà indiquée, que l'on fait réagir avec un dérivé de l'hydrazine de formule (IV) :

$$H_2N\text{-}NH\text{-}R_1 \qquad (IV)$$

pour obtenir un produit de formule (I) dans laquelle R, R' et $R_1$ ont la signification déjà indiquée, que l'on isole et, si désiré, salifie.

L'agent de chloration peut être, par exemple, l'oxychlorure de phosphore ou le chlorure d'oxalyle et de préférence le chlorure de thionyle. On le fait réagir avec le produit de formule (II) de préférence au reflux d'un solvant tel que le benzène, le toluène, le xylène ou le diméthyl formamide.

La réaction du produit de formule (III) avec le dérivé de l'hydrazine de formule (IV) a lieu de préférence au reflux d'un solvant à point d'ébullition de l'ordre de grandeur de celui du xylène ou du diméthyl formamide et tout particulièrement au reflux de l'anisole.

Les produits de départ de formule (II) sont connus ou peuvent être préparés comme indiqué dans J.Chem.Soc. (1961) 2828 et J.Chem.Soc. Suppl.I (1964) 5659. Ceux de formule (IV) sont connus ou peuvent être préparés comme indiqué dans Liebigs Ann. der.Chem.(1931),486, p.95 ou J. Chem. Soc.(1959), 3830-34.

Certains dérivés de formule (I) présentent un caractère basique. On peut avantageusement dans ce cas préparer les sels d'addition des dérivés de formule (I) en faisant réagir, en proportions sensiblement stoechiométriques, un acide minéral ou organique avec ledit dérivé de formule (I). Les sels peuvent être préparés sans isoler les bases correspondantes.

Les dérivés, objet de la présente invention, possèdent de très intéressantes propriétés pharmacologiques ; ils sont doués notamment de remarquables propriétés anxiolytiques.

Ces propriétés sont illustrées plus loin dans la partie expérimentale.

Ces propriétés justifient l'utilisation des dérivés de la pyrazolo/4,3-c/ cinnolin-3-one, ainsi que de leurs sels pharmaceutiquement acceptables, à titre de médicaments.

La présente demande a ainsi également pour objet l'application, à titre de médicaments, des dérivés de la pyrazolo/4,3-c/ cinnolin-3-one, tels que définis par la formule (I) ainsi que de leurs sels d'addition avec les acides pharmaceutiquement acceptables.

Parmi les médicaments, objet de l'invention, on retient de préférence, les médicaments caractérisés en ce qu'ils sont constitués par les nouveaux dérivés de la pyrazolo/4,3-c/ cinnolin-3-one répondant

à la formule (I), dans laquelle R en position 7 ou 8 représente un atome d'hydrogène, un atome d'halogène, un radical alkyle, linéaire ou ramifié renfermant de 1 à 5 atomes de carbone, un radical alkoxy renfermant de 1 à 5 atomes de carbone, un radical nitro ou trifluorométhyle, R' représente un atome d'hydrogène et $R_1$ représente un radical phényle éventuellement substitué par un radical alkyle linéaire ou ramifié renfermant de 1 à 5 atomes de carbone, par un radical alkoxy renfermant de 1 à 5 atomes de carbone ou par un atome d'halogène ou $R_1$ représente un radical pyridyle ou thiazolyle.

Parmi ceux-ci, on retient notamment ceux répondant à la formule (I), dans laquelle R se trouve en position 8 et $R_1$ représente un radical phényle, ainsi que leurs sels d'addition avec les acides pharmaceutiquement acceptables.

Parmi ces derniers, on retient tout particulièrement la 8-chloro 2,5-dihydro 2-phényl 3H-pyrazolo/4,3-c/ cinnolin-3-one,
la 2,5-dihydro 2-phényl 3H-pyrazolo/4,3-c/ cinnolin-3-one et
la 2,5-dihydro 2-pyrid-2-yl 3H-pyrazolo/4,3-c/ cinnolin-3-one,
ainsi que ses sels d'addition avec les acides pharmaceutiquement acceptables.

Les médicaments selon l'invention trouvent leur emploi, par exemple, dans le traitement des états anxieux tels que l'anxiété chronique, associée ou non à de l'insomnie ou à des troubles du comportement, de l'angoisse chez l'adulte ou l'enfant, ou en complément des traitements par neuroleptiques ou antidépresseurs dans les états psychotiques ou dépressifs.

La dose usuelle, variable selon le dérivé utilisé, le sujet et l'affection en cause peut être, par exemple, de 1 mg à 500 mg par jour. Par voie orale, chez l'homme, le dérivé de l'exemple 1 peut être administré à la dose quotidienne de 2 mg à 200 mg, par exemple pour le traitement de l'anxiété chronique, soit environ de 0,03 mg à 3 mg par kilogramme de poids corporel.

L'invention a enfin pour objet les compositions pharmaceutiques qui renferment au moins un dérivé précité ou l'un de ses sels d'addition avec les acides pharmaceutiquement acceptables, à titre de principe actif.

A titre de médicaments,les dérivés répondant à la formule (I) et leurs sels d'addition avec les acides pharmaceutiquement acceptables peuvent être incorporés dans des compositions pharmaceutiques destinées à la voie digestive ou parentérale.

Ces compositions pharmaceutiques peuvent être, par exemple, solides ou

liquides et se présenter sous les formes pharmaceutiques couramment utilisées en médecine humaine, comme par exemple, les comprimés, simples ou dragéifiés, les gélules, les granulés, les suppositoires, les préparations injectables ; elles sont préparées selon les méthodes usuelles. Le ou les principes actifs peuvent y être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

L'invention s'étend en outre aux produits industriels nouveaux utiles notamment pour la préparation des dérivés répondant à la formule (I), à savoir les produits de formule (III) :

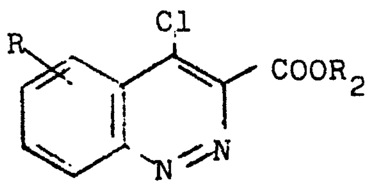

(III)

dans laquelle R et $R_2$ ont la signification déjà indiquée.

Il va être donné maintenant, à titre non limitatif, des exemples de mise en oeuvre de l'invention.

EXEMPLE 1 : 8-chloro-2,5-dihydro-2-phényl 3H-pyrazolo/4,3-c/ cinnolin-3-one.

STADE A :4,6-dichloro 3-cinnoline carboxylate d'éthyle.

On agite pendant 16 heures au reflux une solution de 6 g de 6-chloro 4-hydroxy 3-cinnoline carboxylate d'éthyle dans 300 cm3 de chlorure de thionyle et 1 cm3 de diméthyl formamide, concentre sous pression réduite le chlorure de thionyle en le reprenant plusieurs fois par 50 cm3 de toluène et obtient 6 g d'un résidu verdâtre F. ≃ 50°C.

STADE B :8-chloro-2,5-dihydro-2-phényl 3H-pyrazolo/4,3-c/ cinnolin-3-one.

On mélange sous atmosphère inerte 6 g du produit obtenu au stade précédent à 60 cm3 d'anisole, filtre l'insoluble, ajoute 2,4 cm3 de phényl hydrazine et place le mélange dans un bain à 150°C pendant 2 heures 30 mn. On refroidit, essore, lave à l'acétate d'éthyle, essore à sec, lave avec une solution aqueuse d'acide chlorhydrique N, dissout le solide obtenu dans 100 cm3 d'une solution de 25 cm3 d'ammoniaque concentrée et 75 cm3 d'eau, filtre, extrait à l'éther la phase aqueuse. On précipite le produit attendu à l'aide de phosphate acide de sodium,

purifie en empâtant à l'éthanol, puis à l'eau, sèche et obtient 2,22 g du produit attendu F° > 280°C.

Analyse : $C_{15}$ $H_9$ Cl $N_4O$ = 296,718

Calculé : C% 60,71 H% 3,05 N% 18,88 Cl% 11,94

Trouvé : 60,4 3,1 18,8 11,8.

En opérant selon un procédé analogue à celui de l'exemple 1, on a préparé les produits suivants.

Exemple 2 : 2-(4-chlorophényl)2,5-dihydro 3H-pyrazolo/4,3-c/ cinnolin-3-one.

Exemple 3 : 2,5-dihydro-2-phényl 3H-pyrazolo/4,3-c/ cinnolin-3-one.

Exemple 4 : 2,5-dihydro-2-(4-méthoxyphényl)3H-pyrazolo/4,3-c/ cinnolin-3-one.

Exemple 5 : 2,5-dihydro-2-(4-benzyloxyphényl)3H-pyrazolo/4,3-c/ cinnolin-3-one.

Exemple 6 : 2,5-dihydro-2-(3-trifluorométhylphényl)3H-pyrazolo/4,3-c/ cinnolin-3-one.

Exemple 7 : 2,5-dihydro-2-(2-méthylphényl)3H-pyrazolo/4,3-c/ cinnolin-3-one.

Exemple 8 : Chlorhydrate du 2,5-dihydro-(2-pyridin-2-yl) 3H-pyrazolo /4,3-c/ cinnolin-3-one.

Exemple 9 : Chlorhydrate du 2,5-dihydro-(2-thiazol-2-yl) 3H-pyrazolo /4,3-c/ cinnolin-3-one.

Exemple 10 : Chlorhydrate du 2,5-dihydro-(2-dihydrothiazol-2-yl) 3H-pyrazolo/4,3-c/ cinnolin-3-one.

| Exemple | Sel | R | R' | $R_1$ | Solvant de cristallisation | PF en °C | Formule brute | PM | Microanalyse Calculé Trouvé C% | H% | N% | Cl% |
|---|---|---|---|---|---|---|---|---|---|---|---|---|
| 1 | | 8-Cl | H | 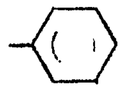 | néant | $>$ 280°C | $C_{15}H_9ClN_4O$ | 296,718 | 60,71<br>60,4 | 3,05<br>3,1 | 18,88<br>18,8 | 11,94<br>11,8 |
| 2 | | H | H | 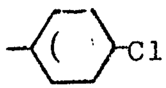 | néant | $>$ 280°C | $C_{15}H_9ClN_4O$ | 296,718 | 60,71<br>60,6 | 3,05<br>3,0 | 18,88<br>18,8 | 11,94<br>12,1 |
| 3 | | H | H | 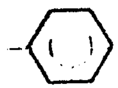 | néant | $>$ 280°C | $C_{15}H_{10}N_4O$ | 262,273 | 68,69<br>68,2 | 3,84<br>3,8 | 21,36<br>21,1 | |
| 4 | | H | H | 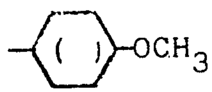 | MeOH | $>$ 280°C | $C_{16}H_{12}N_4O_2$ | 292,30 | 65,74<br>65,5 | 4,13<br>4,1 | 19,16<br>19,0 | |
| 5 | | H | H | 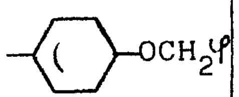 | Isopropanol | $>$ 260°C | $C_{22}H_{16}N_4O_2$ | 368,39 | 71,72<br>71,4 | 4,37<br>4,3 | 15,2<br>14,9 | |
| 6 | | H | H | 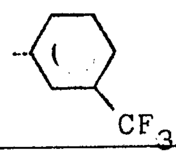 | néant | $>$ 260°C | $C_{16}H_9N_4F_3O$ | 330,27 | 58,18<br>57,8 | 2,74<br>2,7 | 16,96<br>16,8 | F%<br>17,2<br>17,0 |
| 7 | | H | H | 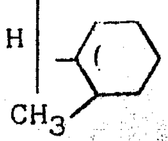 | propanol | $>$ 280°C | $C_{16}H_{12}N_4O$ | 276,30 | 69,55<br>68,85 | 4,37<br>4,32 | 20,27<br>20,0 | |

| Exemple | Sel | R | R' | $R_1$ | Solvant de cristallisation | PF en °C | Formule brute | PM | Microanalyse Calculé/Trouvé C% | H% | N% | Cl% | S% |
|---|---|---|---|---|---|---|---|---|---|---|---|---|---|
| 8 | HCl | H | H | 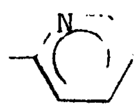 | propanol | > 280°C | $C_{14}H_{10}N_5ClO$ | 299,72 | 56,10<br>55,9 | 3,36<br>3,3 | 23,36<br>23,3 | 11,82<br>11,9 | -<br>- |
| 9 | | H | H | 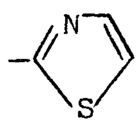 | MeOH | > 280°C | $C_{12}H_7N_5SO$ | 269,286 | 53,52<br>53,1 | 2,62<br>2,6 | 26,00<br>25,6 | -<br>- | 11,9[illegible]<br>11,8[illegible] |
| 10 | HCl | H | H | 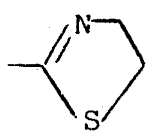 | néant | > 280°C | $C_{12}H_{10}N_5SOCl$ | 307,763 | 46,83<br>47,0 | 3,27<br>3,3 | 22,75<br>22,8 | 11,51<br>10,6 | 10,41<br>10,2 |

Préparation de la 4-chloro 3-cinnolin carboxylate d'éthyle :

On agite pendant 16 heures au reflux une solution de 4 g de 6-chloro 4-hydroxy 3-cinnolin carboxylate d'éthyle dans 250 cm3 de chlorure de thionyle, concentre sous pression réduite, le chlorure de thionyle en le reprenant 3 fois par 50 cm3 de toluène et obtient 4 g d'un résidu verdâtre,(huile).

Exemple 11 :

On a préparé des comprimés répondant à la formule :

- 8-chloro-2,5-dihydro-2-phényl 3H-pyrazolo /4,3-c/ cinnolin-3-one.......................................... 10 mg ;
- Excipient q. s. pour un comprimé terminé à................ 100 mg.

(Détail de l'excipient : lactose, amidon, talc, stéarate de magnésium).

Exemple 12 :

On a préparé des comprimés répondant à la formule :

- 2,5-dihydro-2-phényl 3H-pyrazolo/4,3-c/ cinnolin-3-one.... 10 mg.
- Excipient q.s. pour un comprimé terminé à................. 100 mg.

(Détail de l'excipient : lactose, amidon, talc, stéarate de magnésium).

Exemple 13 :

On a préparé des comprimés répondant à la formule :

- Chlorhydrate 2,5-dihydro-2-pyridin-2-yl) 3H-pyrazolo /4,3-c/ cinnolin-3-one.......................................... 5 mg.
- Excipient q.s. pour un comprimé terminé à................. 100 mg.

(Détail de l'excipient : lactose, amidon, talc, stéarate de magnésium).

Etude pharmacologique.

1) Affinité pour les récepteurs des benzodiazépines.

On homogénéise au vingtième (poids/volume) dans le sucrose 0,32M, les cortex prélevés sur des cerveaux de rats mâles pesant 150 g en moyenne.

Après centrifugation de l'homogénat à 1000 g pendant 10 minutes à 0°C, le surnageant est centrifugé à 30 000 g pendant 20 minutes à +4°C. Le culot est mis en suspension dans 20 volumes de tampon Tris Hcl 50mM pH 7,4 et centrifugé à 30 000 g pendant 20 minutes à +4°C. Le nouveau culot obtenu est mis en suspension dans 50 ml de tampon Krebs Tris HCl pH 7,4.

On fait ensuite incuber pendant 30 minutes à 0°C, 2 ml de suspension en présence de $^{3}H$ diazepam à la concentration $10^{-9}M$, seul, avec des concentrations croissantes du produit à tester, ou, pour déterminer la fixation non spécifique, avec du diazepam non radioactif à la concentration $10^{-6}M$.

Les suspensions incubées sont filtrées sur Whatman GF/C et les filtres

sont lavés par deux fois 5 ml de tampon Krebs Tris HCl pH 7,4 à 0°C.

La radioactivité des filtres est mesurée par scintillation liquide.

L'activité du produit est exprimée en C. I. 50 : concentration inhibant 50 % de la liaison spécifique de $^{3}H$ diazepam déterminée graphiquement.

Résultats :

| Produit de l'exemple | CI 50 en nM |
|---|---|
| 1 | 1,4 |
| 2 | 3,7 |
| 3 | 0,9 |
| 4 | 2,5 |
| 5 | 18 |
| 8 | 3,1 |
| 9 | 3,1 |

2) Test de l'escalier.

L'appareil et le protocole utilisés ont été décrits par THIEBOT et coll. (Psychopharmacologia (Berlin) 1973, 31, 77).

L'expérimentation est réalisée sur des lots de 15 rats naïfs. Les animaux sont déposés individuellement dans l'enceinte 1 heure après l'administration du composé étudié. Le nombre de redressements effectués et le nombre de marches montées sont comptés pendant 3 mn.

A la dose de 20 mg/Kg, le produit de l'exemple 1 diminue de 46 % le nombre de redressements, sans modification notable du nombre de marches montées. Il présente donc une bonne activité anxiolytique.

3) Test des 4 plaques.

L'appareil (Apelab) et le protocole utilisés ont été décrits par BOISSIER et coll. (European J. Pharmacol. 1968, 4, 145). Les plaques sont reliées à un stimulateur (U. Sachs, Roucaire) qui permet de délivrer des chocs électriques de 120 volts pendant 0,5 s. Les essais sont réalisés sur des lots de 10 souris ou rats 1/2 h après administration per os du composé étudié. Chaque animal est déposé individuellement sur l'appareil. Après 15s d'exploration libre , il est soumis à un choc électrique chaque fois qu'il passe d'une plaque à l'autre, un minimum de 3 secondes étant observé entre deux chocs. Le nombre de chocs délivrés est compté pendant 1 minute. Les résultats obtenus sont comparés à ceux observés chez des animaux témoins par un test de Dunnett.

A la dose de 20 mg/Kg de produit de l'exemple 1, le nombre de chocs délivrés est augmenté de 63 % chez les souris, et de 41 % chez les rats.

L'augmentation du nombre de chocs est de 50 % chez les souris à la dose de 50 mg/kg pour le produit de l'exemple 3 et à la dose de 100 mg/kg pour le produit de l'exemple 8.

Le produit des exemples 1, 3 et 8 présentent donc une bonne activité anxiolytique.

4) Etude de la toxicité aigüe.

On a évalué la dose létale $DL_0$ du composé de l'exemple 1 après administration par voie orale chez la souris.

On appelle $DL_0$ la dose maximale ne provoquant aucune mortalité en 8 jours.

Les résultats sont les suivants :

| Produit de l'exemple | DLO |
|---|---|
| 1 | > 400 |
| 3 | > 200 |
| 4 | > 400 |
| 5 | 400 |
| 6 | 400 |
| 7 | 200 |
| 8 | > 400 |
| 10 | > 400 |

0187551

Revendications :

1) Les dérivés de la pyrazolo /4,3-c/ cinnolin-3-one, ainsi que leurs sels d'addition avec les acides minéraux ou organiques, caractérisés en ce qu'ils répondent à la formule générale (I) :

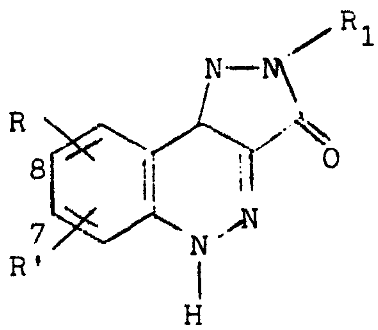

(I)

dans laquelle R en position 7 ou 8 représente un atome d'hydrogène, un atome d'halogène, un radical alkyle linéaire ou ramifié renfermant de 1 à 5 atomes de carbone, un radical alkoxy renfermant de 1 à 5 atomes de carbone, un radical nitro ou trifluorométhyle ou ensemble avec R' forme un pont alkylène $C_1$-$C_4$ dioxy en 7-8, R' représente un atome d'hydrogène ou ensemble avec R représente un pont alkylène $C_1$-$C_4$ dioxy en 7-8 et $R_1$ représente un radical phényle, pyridyle, thiazolyle, dihydrothiazolyle ou thiényle éventuellement substitués par un radical alkyle linéaire ou ramifié renfermant de 1 à 5 atomes de carbone, par un radical alkoxy renfermant 1 à 5 atomes de carbone ou par un atome d'halogène, par un groupement mono-, di- ou tri-halogéno méthyle ou par un groupement aralkyloxy renfermant de 7 à 12 atomes de carbone.

2) Les dérivés répondant à la formule (I) selon la revendication 1, ainsi que leurs sels d'addition avec les acides minéraux ou organiques, caractérisés en ce que, dans ladite formule (I) :
R en position 7 ou 8 représente un atome d'hydrogène, un atome d'halogène, un radical alkyle linéaire ou ramifié renfermant de 1 à 5 atomes de carbone, un radical alkoxy renfermant de 1 à 5 atomes de carbone, un radical nitro ou trifluorométhyle, R' représente un atome d'hydrogène et $R_1$ représente un radical phényle éventuellement substitué par un radical alkyle linéaire ou ramifié renfermant de 1 à 5 atomes de carbone, par un radical alkoxy renfermant de 1 à 5 atomes de carbone, ou par un atome d'halogène, ou $R_1$ représente un radical pyridyle ou thiazolyle.

3) Les dérivés selon la revendication 2, ainsi que leurs sels d'addition avec les acides minéraux ou organiques, caractérisés en ce que, dans ladite formule (I), R se trouve en position 8 et $R_1$ représente un radical phényle.

4) Les dérivés répondant à la formule (I) dont les noms suivent :

- la 8-chloro 2,5-dihydro-2-phényl 3H-pyrazolo/4,3-c/ cinnolin-3-one,
- la 2,5-dihydro 2-phényl 3H-pyrazolo/4,3-c/ cinnolin-3-one,
- la 2,5-dihydro 2-pyrid-2-yl 3H-pyrazolo/4,3-c/ cinnolin-3-one ainsi que leurs sels d'addition avec les acides mineraux ou organiques.

5) Procédé de préparation des dérivés de formule (I) telle que définie à la revendication 1, ainsi que de leurs sels, caractérisé en ce que l'on fait réagir un produit de formule (II) :

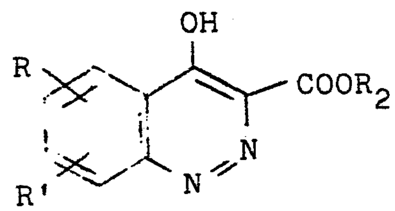

(II)

dans laquelle R et R' ont la signification déjà indiquée, et $R_2$ représente un radical alkyle linéaire renfermant de 1 à 5 atomes de carbone avec un agent de chloration, pour obtenir un produit de formule (III) :

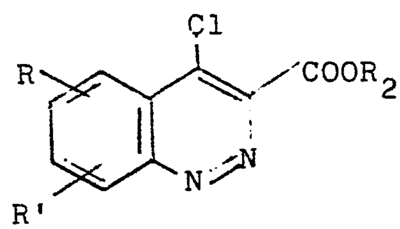

(III)

dans laquelle R, R' et $R_2$ ont la signification déjà indiquée, que l'on fait réagir avec un dérivé de l'hydrazine de formule (IV) :

$$H_2N - NH - R_1 \qquad (IV)$$

pour obtenir un produit de formule (I) dans laquelle R, R' et $R_1$ ont la signification déjà indiquée, que l'on isole et, si désiré, salifie.

6) Procédé selon la revendication 5, caractérisé en ce que :
- la réaction du produit de formule (II) avec l'agent de chloration est effectuée au reflux dans le chlorure de thionyle en présence de diméthyl formamide ;
- la réaction du produit de formule (III) avec le dérivé de l'hydrazine est effectuée au reflux dans l'anisole.

7) Médicaments, caractérisés en ce qu'ils sont constitués par les dérivés de la pyrazolo /4,3-c/ cinnolin-3-one tels que définis par la formule (I) de la revendication 1, ainsi que par leurs sels d'addition avec les acides pharmaceutiquement acceptables.

8) Médicaments caractérisés en ce qu'ils sont constitués par les

dérivés de la pyrazolo /4,3-c/ cinnolin-3-one tels que définis à l'une quelconque des revendications 2,3 ou 4 ainsi que par leurs sels d'addition avec les acides pharmaceutiquement acceptables.

9) Compositions pharmaceutiques, caractérisées en ce qu'elles renferment, à titre de principe actif, l'un au moins des médicaments tels que définis à l'une des revendications 7 ou 8.

10) A titre de produits industriels nouveaux, les produits de formule (III) :

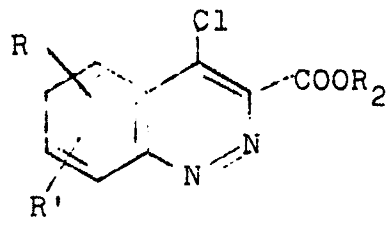

dans laquelle R, R' et $R_2$ ont la signification déjà indiquée.

Office européen des brevets

# RAPPORT DE RECHERCHE EUROPEENNE

0187551
Numero de la demande

EP 85 40 0024

## DOCUMENTS CONSIDERES COMME PERTINENTS

| Catégorie | Citation du document avec indication, en cas de besoin des parties pertinentes | Revendication concernee | CLASSEMENT DE LA DEMANDE (Int. Cl.4) |
|---|---|---|---|
| A | EP-A-0 022 078 (CIBA-GEIGY)<br>* Revendications 1,12 *<br>--- | 1,7 | C 07 D 487/04<br>C 07 D 491/14<br>A 61 K 31/50<br>C 07 D 237/28 //<br>(C 07 D 487/04<br>C 07 D 237:00<br>C 07 D 231:00 ) |
| A | GB-A-2 131 801 (CIBA-GEIGY)<br>* Revendication 1; page 2, lignes 34-43 *<br>--- | 1,7 | |
| X | GB-A-1 474 399 (I.C.I.)<br>* Page 2, formule 4; exemples *<br>----- | 10 | |
| | | | DOMAINES TECHNIQUES RECHERCHES (Int. Cl.4) |
| | | | C 07 D 487/00<br>A 61 K 31/00<br>C 07 D 237/00 |

Le présent rapport de recherche a été établi pour toutes les revendications

| Lieu de la recherche | Date d'achèvement de la recherche | Examinateur |
|---|---|---|
| LA HAYE | 02-09-1985 | ALFARO I. |

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

OEB Form 1503. 03.82